(19) Europäisches Patentamt
European Patent Office
Office européen des brevets

(11) Veröffentlichungsnummer: **0 005 143**
**B1**

(12) # EUROPÄISCHE PATENTSCHRIFT

(45) Veröffentlichungstag der Patentschrift: **14.07.82**

(51) Int. Cl.[3]: **C 07 D 207/27**, A 61 K 31/40 // C07D207/38, C07C103/84

(21) Anmeldenummer: **79100379.1**

(22) Anmeldetag: **09.02.79**

(54) **1-Benzoyl-2-pyrrolidinonderivat, Verfahren zu seiner Herstellung und dieses enthaltende Arzneimittel.**

(30) Priorität: **10.02.78 CH 1403/78**
**22.11.78 CH 11981/78**

(43) Veröffentlichungstag der Anmeldung: **14.11.79 Patentblatt 79/23**

(45) Bekanntmachung des Hinweises auf die Patenterteilung: **14.07.82 Patentblatt 82/28**

(84) Benannte Vertragsstaaten: **BE CH DE FR GB IT LU NL SE**

(56) Entgegenhaltungen:
**DE-A-2 613 [illegible]**
**Chemical Abstracts vol. 58, no. 6, 18 March 1963, Columbus, Ohio, USA F. KORTE et al »Acyl-lacton rearrangement, XXII. Rearrangement of 3-aroylpyrrolidones in concentrated hydrochloric acid to pyrrolidine derivatives« Spalte 5736 d.**

(73) Patentinhaber: **F. HOFFMANN-LA ROCHE & CO. Aktiengesellschaft, CH-4002 Basel (CH)**

(72) Erfinder: **Kyburz, Emilio, Dr., Unterer Rebbergweg 127, CH-4153 Reinach (CH)**
Erfinder: **Aschwanden, Werner, Grellingerstrasse 4, CH-4107 Ettingen (CH)**

(74) Vertreter: **Lederer, Franz, Dr. et al, Patentanwälte Lederer, Meyer Lucile-Grahn-Strasse 22, D-8000 München 80 (DE)**

EP 0 005 143 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

d) 4-(p-Methoxybenzoylamino)buttersäure cyclisiert oder
e) eine Verbindung der allgemeinen Formel

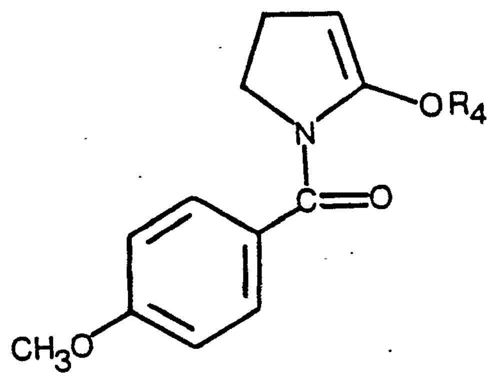

III

worin $R_4$ niederes Alkyl bedeutet,

hydrolysiert.

Nach einem ersten Aspekt des erfindungsgemäßen Verfahrens kann die Verbindung der Formel I demgemäß dadurch hergestellt werden, daß man 2-Pyrrolidinon in 1-Stellung entsprechend acyliert, d. h. das Wasserstoffatom in 1-Stellung des 2-Pyrrolidinons durch einen p-Methoxybenzoylrest ersetzt. Hierbei kann man Methoden verwenden, welche für derartige Acylierungen an sich allgemein bekannt sind. Als Acylierungsmittel verwendet man ein hinreichend reaktionsfähiges Derivat der p-Methoxybenzoesäure, insbesondere ein reaktionsfähiges Halogenid dieser Säure (vorzugsweise p-Methoxybenzoylchlorid), reaktionsfähige Ester, beispielsweise Polyhalophenylester, dieser Säure (wie p-Methoxybenzoesäure-pentachlorphenylester) u. dgl.

Bei der Acylierung von 2-Pyrrolidinon mit p-Methoxybenzoylchlorid arbeitet man zweckmäßigerweise in Gegenwart eines inerten organischen Lösungsmittels und einer Base. Hierbei eignen sich als Lösungsmittel in erster Linie Äther (wie Diäthyläther, Tetrahydrofuran, Dioxan usw.), aromatische Kohlenwasserstoffe (wie Toluol usw.) oder dergleichen und als Basen tertiäre Amine, wie Triäthylamin oder dergleichen; die Acylierung kann auch in Pyridin durchgeführt werden, welches gleichzeitig als Lösungsmittel und als Base fungiert. Weder die Anwesenheit eines Lösungsmittels noch die Anwesenheit einer Base ist indessen zwingend; es ist durchaus möglich, die Komponenten zusammen während etwa einer Stunde auf etwa 80–90°C zu erhitzen oder in einem geeigneten Lösungsmittel (z. B. in einem Äther, einem aromatischen Kohlenwasserstoff od. dgl.) während einiger Stunden am Rückfluß zu erhitzen. Weiterhin ist es möglich, das 2-Pyrrolidinon zunächst mit einer zur Abstraktion des Wasserstoffatoms am Stickstoffatom in 1-Stellung befähigten Base zu behandeln und hierauf mit p-Methoxybenzoylchlorid reagieren zu lassen. Bei dieser Verfahrensvariante kann als Base beispielsweise ein Alkalimetallhydrid, wie Natriumhydrid oder dergleichen und als Lösungsmittel ein aromatischer Kohlenwasserstoff (wie Benzol), Dimethylformamid oder dergleichen verwendet werden. Es ist auch möglich das 2-Pyrrolidinon in Form eines reaktionsfähigen Derivates einzusetzen, in welchem am Stickstoffatom in 1-Stellung eine leicht abspaltbare Gruppe sitzt, beispielsweise eine leicht abspaltbare metallorganische Gruppe, insbesondere eine Trialkylsilylgruppe; ein bevorzugtes derartiges Derivat ist das 1-Trimethylsilyl-2-pyrrolidinon.

Nach einem zweiten Aspekt des erfindungsgemäßen Verfahrens kann die Verbindung der Formel I dadurch hergestellt werden, daß man 1-(p-Hydroxybenzoyl)-2-pyrrolidinon methyliert. Bei diesem Verfahrensaspekt handelt es sich also um eine Methylierung einer phenolischen Hydroxylgruppe, und man kann hierbei Methoden verwenden, welche für derartige Methylierungen phenolischer Hydroxylgruppen an sich allgemein bekannt sind.

Als Methylierungsmittel eignen sich beispielsweise Dimethylsulfat, Methyljodid oder dergleichen. Bei Verwendung derartiger Methylierungsmittel arbeitet man zweckmäßigerweise in Gegenwart einer Base, wie Natriummethylat, Natriumhydrid oder dergleichen und in Gegenwart eines unter den Reaktionsbedingungen inerten organischen Lösungsmittels, beispielsweise in Dimethylformamid, einem aromatischen Kohlenwasserstoff, wie Benzol, Toluol und dergleichen. Auch das Diazomethan ist ein geeignetes Methylierungsmittel. Bei Verwendung von Diazomethan arbeitet man zweckmäßigerweise in ätherischer Lösung, z. B. in Tetrahydrofuran, Diäthyläther u. dgl. oder in Gemischen solcher Äther. Das als Ausgangsmaterial verwendete 1-(p-Hydroxybenzoyl)-2-pyrrolidinon kann beispielsweise dadurch hergestellt werden, daß man das Wasserstoffatom am 1-ständigen Stickstoffatom des 2-Pyrrolidinons durch einen p-Benzyloxybenzoylrest ersetzt (beispielsweise mittels p-Benzyloxybenzoylchlorid in Analogie zu den vorstehend im Zusammenhang mit dem ersten Verfahrensaspekt beschriebenen Methoden), worauf man das erhaltene 1-(p-Benzoyloxybenzoyl)-2-pyrrolidinon nach an sich bekannten Methoden debenzyliert (beispielsweise durch Hydrierung in Gegenwart eines geeigneten Katalysators, wie Palladium).

der Herstellung der Verbindung der allgemeinen Formel III in einem mit Wasser nicht mischbaren Lösungsmittel arbeitet, so überwiegt bei der Hydrolyse die Bildung des gewünschten Produkts, d. h. der Verbindung der Formel I. Die Verbindung der Formel III wird — wie gesagt — nicht gefaßt, sondern direkt hydrolysiert, wobei die Hydrolyse in an sich bekannter Weise durch Zugabe von Wasser, von wäßrigem Alkali (z. B. Lithiumhydroxydlösung) oder wäßriger Säure (z. B. wäßriger Salzsäure) erfolgen kann.

Wie eingangs erwähnt, ist das 1-(p-Methoxybenzoyl)-2-pyrrolidinon der Formel I eine neue Verbindung mit wertvollen pharmakodynamischen Eigenschaften. Die Verbindung der Formel I weist nur eine sehr geringe Toxizität auf, und es hat sich gezeigt, daß sie in den nachstehend beschriebenen Tierversuchen experimentell auf verschiedene Weise hervorgerufener cerebraler Insuffizienz entgegenzuwirken vermag.

## A) Posthypercapnische »Avoidance«-Acquisition

Die Test-Apparatur ist eine »shuttle box« mit einer 10 cm hohen Hürde in der Mitte und elektrifizierbarem Gitterboden. In der schalldichten Kammer ist ein Lautsprecher montiert. Eine oder drei Stunden nach Verabreichung von Kontroll- oder Präparat-Injektion werden unerfahrene Ratten (120 – 150 g; 10 pro Gruppe) für 12 Sekunden in reines $CO_2$-Milieu gebracht. Eine dritte Gruppe von 10 Ratten wird weder mit dem Präparat noch mit $CO_2$ behandelt. Drei Minuten nach $CO_2$-Behandlung müssen die Ratten aller drei Gruppen in der »shuttle box« in folgendem Programm einen bedingten und unbedingten Reflex erlernen: 10 Sek. Stille — 5 Sek. Ton (»avoidance response«) — 15 Sek. Ton + Fuß-Schock (»escape response«); sechsmal hintereinander. Für jeden der sechs Einzelversuche wird die Reaktionszeit (Zeit bis die Ratte über die Hürde springt) jeder Ratte gemessen und die statistische Signifikanz der Unterschiede zwischen den verschiedenen Gruppen mittels Rang-Test berechnet.

Als »aktiv« bezeichnet man diejenige Dosis eines Präparats, welche während der sechs Einzelversuche eine signifikante Wirkung zeigt; dabei müssen die mit dem Präparat und $CO_2$ behandelten Tiere signifikant besser lernen als die nur mit $CO_2$ behandelten Tiere und gleich gut wie die weder mit dem Präparat noch mit $CO_2$ behandelten Tiere.

## B) »Passive Avoidance«-Test mit Elektroschock-Amnesie

Die Testapparatur ist eine Skinnerbox mit elektrifizierbarem Gitterboden und einer grauen Viereckplattform in einer Ecke. Unerfahrene männliche Ratten, 100 – 120 g schwer, werden auf die graue Plattform gesetzt. Jedesmal wenn sie auf den Gitterboden hinuntersteigen, erhalten sie einen Elektroschock. Nach 3 – 5 Versuchen zeigen die Ratten eine sogenannte »passive avoidance response« d. h. Weigerung von der Plattform herunterzusteigen. Unmittelbar nach Acquisition der Weigerung werden drei Gruppen zu je 20 Ratten gebildet. Eine Gruppe erhält einen Elektroschock (45 mA, 2 Sek.) durch die Ohren und eine i.p. NaCl-Injektion. Die zweite Gruppe erhält einen Elektroschock durch die Ohren und eine i.p. Injektion der Testsubstanz. Die dritte Gruppe erhält nur NaCl. Nach drei Stunden wird jede Ratte einmal auf die Plattform gesetzt und die Verweildauer (max. 60 Sek.) gemessen. Die signifikante Wirkung der Testsubstanz im Vergleich zu den beiden Kontrollgruppen wird mittels Rang-Test berechnet. Als »aktiv« bezeichnet man diejenige Dosis eines Präparates, welche eine signifikante Schutzwirkung gegen den Elektroschock zeigt (die mit aktiver Dosis eines Präparats und Elektroschock behandelten Tiere zeigen eine lange Verweildauer, ebenso die nicht mit Elektroschock behandelten Tiere, wogegen die mit NaCl und Elektroschock behandelten Tiere eine kurze Verweildauer zeigen).

## C) Verzögerung des Haloperidol-induzierten »knock out« in einem »Continuous Avoidance«-Programm mit Totenkopfaffen

Männliche, erwachsene Totenkopfaffen (Saimiri sciureus), 0,6 bis 1,2 kg schwer, einzeln gehalten, werden in einer 2-Tasten Skinnerbox auf folgendes »continuous avoidance«-Programm trainiert: »Avoidance-shock«-Intervall 40 Sek.; »shock-shock«-Intervall 20 Sek.; Fuß-Schock max. 5 Sek. Affen mit regelmäßiger Grundleistung erhalten Haloperidol 1,0 mg/kg p.o. zur Bestimmung der »knock-out«-Zeit (Blockierung von »avoidance« und »escape«). Affen mit stabilen »knock-out«-Zeiten werden für die Evaluation von Versuchspräparaten als potentielle cerebrale Insuffizienzverbesserer selektioniert. Die Präparate können zu verschiedenen Zeiten vor der Behandlung mit Haloperidol injiziert werden. Als »aktiv« bezeichnet man diejenige Dosis eines Präparates, welche bei Verabreichung vor der Behandlung mit Haloperidol eine signifikante Verzögerung des »knock-out«-Zeitpunkts in einem Drei-Stunden-Test bewirkt.

Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 10 bis 2500 mg 1-(p-Methoxybenzoyl)-2-pyrrolidinon angemessen sein, wobei aber die soeben angegebene obere Grenze im Hinblick auf die geringe Toxizität des 1-(p-Methoxybenzoyl)-2-pyrrolidinons ohne weiteres überschritten werden kann, wenn sich dies als angezeigt erweisen sollte.

In den nachfolgenden Beispielen, welche die vorliegende Erfindung illustrieren, ihren Umfang jedoch in keiner Weise einschränken sollen, sind sämtliche Temperaturen in Celsiusgraden angegeben.

## Beispiel 1

40,0 g p-Methoxybenzoylchlorid, 25,0 g 2-Pyrrolidinon und 110 ml abs. Diäthyläther werden zwischen 0 bis 10° unter Rühren mit 52,5 ml Triäthylamin versetzt. Man rührt weitere 30 Minuten bei Zimmertemperatur und 3 Stunden am Rückfluß, kühlt dann ab und versetzt bei 2° mit kaltem Wasser. Die unlöslichen Anteile werden abgesaugt und mit Wasser und Diäthyläther gewaschen. Der so erhaltene Festkörper wird nach Trocknen über Phosphorpentoxid aus Alkohol umkristallisiert. Man erhält 1-(p-Methoxybenzoyl)-2-pyrrolidinon, welches bei 121 – 122° schmilzt.

## Beispiel 2

Zu einer Lösung von 37,0 g p-Methoxybenzoylchlorid in 50 ml Dimethylformamid werden bei –10° 20,2 g des mittels Natriumhydrid hergestellten Natriumsalzes von 2-Pyrrolidinon, suspendiert in 270 ml Dimethylformamid, in 4 Portionen zugegeben. Anschließend wird 1 Stunde bei Raumtemperatur und dann 4 Stunden bei 40° gerührt. Das Lösungsmittel wird abgedampft und der Rückstand mit Diäthyläther und mit kalter Natriumbicarbonatlösung versetzt. Die unlöslichen kristallinen Anteile werden abfiltriert, mit Wasser und Diäthyläther gewaschen und über Phosphorpentoxid im Vakuum getrocknet. Man erhält 1-(p-Methoxybenzoyl)-2-pyrrolidinon vom Schmelzpunkt 120 – 121°.

## Beispiel 3

20 g p-Methoxybenzoylchlorid und 20 g 2-Pyrrolidinon werden in 20 ml Diäthyläther 16 Stunden am Rückfluß gekocht, wonach zum Reaktionsgemisch Diäthyläther, Eis und 2 N wässeriger Ammoniak zugegeben werden. Die unlöslichen Anteile werden abfiltriert und mit Diäthyläther und Wasser ionenfrei gewaschen. Der Filterkuchen wird getrocknet, und man erhält 1-(p-Methoxybenzoyl)-2-pyrrolidinon vom Schmelzpunkt 119,5 – 120,5°.

## Beispiel 4

Man arbeitet wie in Beispiel 3 angegeben, erhitzt aber die Komponenten nicht in Diäthyläther, sondern in 20 ml Toluol. Das erhaltene 1-(p-Methoxybenzoyl)-2-pyrrolidinon schmilzt bei 117 – 118°.

## Beispiel 5

10 g 2-Pyrrolidinon und 10 g p-Methoxybenzoylchlorid werden ohne Lösungsmittel während 1 Stunde auf 80 – 90° (Innentemperatur) erwärmt. Hierauf läßt man abkühlen und arbeitet wie in Beispiel 3 beschrieben auf. Nach Umkristallisieren aus Alkohol erhält man 1-(p-Methoxybenzoyl)-2-pyrrolidinon vom Schmelzpunkt 120 – 121°.

## Beispiel 6

24,4 g p-Methoxybenzoylchlorid und 22,5 g 1-Trimethylsilyl-2-pyrrolidinon werden vermischt, worauf man bei Raumtemperatur 10 Minuten rührt. Hierauf wird in einem Ölbad von 80° das entstandene Trimethylchlorsilan unter vermindertem Druck abdestilliert. Der Rückstand wird mit 100 ml Diäthyläther verrührt. Man filtriert ab und kristallisiert den Filterkuchen aus Äthylalkohol um. Man erhält 1-(p-Methoxybenzoyl)-2-pyrrolidinon vom Schmelzpunkt 120 – 121°.

3 : 1) umkristallisiert. Man erhält 4-(p-Methoxybenzoylamino)buttersäure vom Schmelzpunkt 120,5 – 122°.

10 g Phosphorpentoxid und 6 ml ortho-Phosphorsäure (mindestens 85%ig) werden miteinander erwärmt. Zu der entstehenden Lösung werden bei Raumtemperatur 2,0 g 4-(p-Methoxybenzoylamino)-buttersäure zugegeben. Das Reaktionsgemisch wird 60 Minuten auf 50° erwärmt, anschließend mit Eis versetzt und mit Essigester extrahiert. Die organische Phase wird zunächst mit kaltem Wasser, dann mit kalter Natriumbicarbonatlösung und schließlich nochmals mit Wasser gewaschen und über Natriumsulfat getrocknet. Der Rückstand wird mit Diäthyläther verrührt, und man erhält 1-(p-Methoxybenzoyl)-2-pyrrolidinon vom Schmelzpunkt 120 – 122°.

## Beispiel 11

20,0 g 4-(p-Methoxybenzoylamino)buttersäure, 50 ml Toluol und 9,2 ml Thionylchlorid werden 2 Stunden am Rückfluß erwärmt, worauf man mit Entfärbungskohle behandelt und eindampft. Der Rückstand wird in Methylenchlorid gelöst und über neutralem Aluminiumoxid chromatographiert. Das mit Methylenchlorid eluierte 1-(p-Methoxybenzoyl)-2-pyrrolidinon wird aus Alkohol umkristallisiert und zeigt einen Schmelzpunkt von 119 – 120°.

## Beispiel 12

276 mg Lithiumhydrid (98%ig), 40 ml Benzol und 4,2 g 2-Methoxypyrrolin werden 2 Stunden unter Stickstoff und unter gutem Rühren am Rückfluß gekocht. Nach Abkühlen auf 25° werden 6,6 g p-Methoxybenzoylchlorid, gelöst in 25 ml Benzol, zugegeben, worauf 2 Stunden unter Rückfluß und 16 Stunden bei Raumtemperatur gerührt wird. Zum Reaktionsgemisch werden dann 100 ml Essigester und danach 3,0 g Lithiumhydroxid (98%ig), gelöst in 25 ml Wasser, innerhalb von 15 Minuten bei Raumtemperatur zugegeben. Die organische Phase wird mit Wasser neutral gewaschen, über Natriumsulfat getrocknet und unter vermindertem Druck eingedampft. Nach Umkristallisieren aus Äthylalkohol erhält man 1-(p-Methoxybenzoyl)-2-pyrrolidon vom Schmelzpunkt 119 – 120°.

## Beispiel 13

4,2 g 2-Methoxypyrrolin werden in 25 ml Benzol gelöst und bei Raumtemperatur mit 6,6 g p-Methoxybenzoylchlorid, gelöst in 30 ml Benzol, versetzt. Man rührt 60 Minuten bei Raumtemperatur und 4 Stunden bei Rückflußtemperatur, worauf 30 ml Benzol zugegeben werden und das Reaktionsgemisch weitere 24 Stunden am Rückfluß gekocht wird. Die flüchtigen Anteile werden abdestilliert; der Rückstand wird in Äthylalkohol aufgenommen, worauf man einengt, den Rückstand mit Diäthyläther verrührt, filtriert und den Filterkuchen in Essigester aufnimmt. Die Essigesterlösung wird mit Natriumbicarbonatlösung und Wasser gewaschen, über Natriumsulfat getrocknet und eingeengt. Der Rückstand wird im Vakuum sublimiert, und man erhält 1-(p-Methoxybenzoyl)-2-pyrrolidinon vom Schmelzpunkt 119 – 120°.

## Beispiel 14

1-(p-Methoxybenzoyl)-2-pyrrolidinon wird als Wirkstoff zur Herstellung von Tabletten folgender Zusammensetzung verwendet:

| | pro Tablette |
|---|---|
| Wirkstoff (feingemahlen) | 100 mg |
| Milchzucker (pulverisiert) | 150 mg |
| Maisstärke (weiß) | 230 mg |
| Polyvinylpyrrolidon | 15 mg |
| Magnesiumstearat | 5 mg |
| | 500 mg |

Der feingemahlene Wirkstoff, der pulverisierte Milchzucker und ein Teil der weißen Maisstärke werden miteinander vermischt. Die Mischung wird gesiebt, worauf man sie mit einer Lösung von Polyvinylpyrrolidon in Wasser gefeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

Beispiel 18

1-(p-Methoxybenzoyl)-2-pyrrolidinon wird als Wirkstoff zur Herstellung von Doppelampullen folgender Zusammensetzung verwendet:

| | | |
|---|---|---|
| Wirkstofflösung | | |
| Wirkstoff | | 100 mg |
| Glycofurol | ad | 3,5 ml |
| Diluens | | |
| Natriumchlorid | | 67,5 mg |
| Wasser zu Injektionszwecken | ad | 7,5 ml |

Vor der Injektion ist das Diluens dem Inhalt der Wirkstoffampullen zuzufügen. Man erhält 10 ml einer spritzfertigen Lösung, enthaltend 100 mg Wirkstoff.

Beispiel 19

1-(p-Methoxybenzoyl)-2-pyrrolidinon wird als Wirkstoff zur Herstellung von Doppelampullen folgender Zusammensetzung verwendet:

| | | |
|---|---|---|
| Wirkstofflösung | | |
| Wirkstoff | | 100 mg |
| Polyäthylenglykol | | 1,5 ml |
| Glycofurol | ad | 4 ml |
| Diluens | | |
| Wasser zu Injektionszwecken | | 6 ml |

Vor der Injektion ist das Diluens dem Inhalt der Wirkstoffampullen zuzufügen. Man erhält 10 ml einer spritzfertigen Lösung, enthaltend 100 mg Wirkstoff.

**Patentansprüche**

1. 1-(p-Methoxybenzoyl)-2-pyrrolidinon.
2. 1-(p-Methoxybenzoyl)-2-pyrrolidinon zur Verwendung als pharmazeutischer Wirkstoff.
3. 1-(p-Methoxybenzoyl)-2-pyrrolidinon zur Verwendung bei der Bekämpfung bzw. Verhütung cerebraler Insuffizienz bzw. bei der Verbesserung der intellektuellen Leistungsfähigkeit.
4. Verfahren zur Herstellung von 1-(p-Methoxybenzoyl)-2-pyrrolidinon, dadurch gekennzeichnet, daß man

a) 2-Pyrrolidinon in 1-Stellung entsprechend acyliert oder
b) 1-(p-Hydroxybenzoyl)-2-pyrrolidinon methyliert oder
c) ein 2-Pyrrolinon-Derivat der allgemeinen Formel

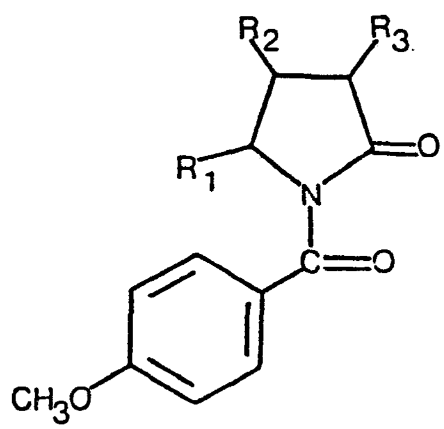

II

worin eines von $R_1$ und $R_3$ Wasserstoff und das andere zusammen mit $R_2$ eine zweite C–C-Bindung bedeutet,

or a mixture of the two compounds falling under general formula II, or

d) cyclizing 4-(p-methoxybenzoylamino)butyric acid, or
e) hydrolyzing a compound of the general formula

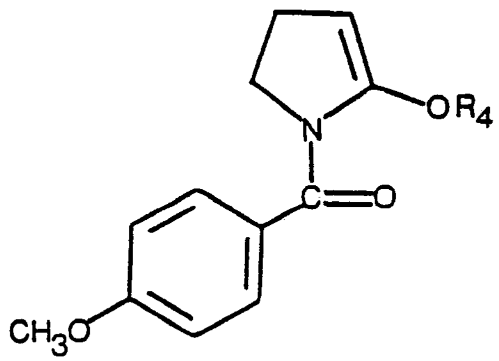

III

wherein $R_4$ signifies lower alkyl.

5. Process according to claim 4, characterized in that 2-pyrrolidinone in the form of its 1-trimethylsilyl derivative is reacted with p-methoxybenzoyl chloride or in that 2-pyrrolidinone is reacted with p-methoxybenzoic acid pentachlorophenyl ester or in that 1-(p-hydroxybenzoyl)-2-pyrrolidinone is reacted with diazomethane.
6. Medicament, containing 1-(p-methoxybenzoyl)-2-pyrrolidinone.
7. Medicament containing 1-(p-methoxybenzoyl)-2-pyrrolidinone for use in the control or prevention of cerebral insufficiency or in the improvement of intellectual capacity.

**Revendications**

1. 1-(p-méthoxybenzoyl)-2-pyrrolidinone.
2. 1-(p-méthoxybenzoyl)-2-pyrrolidinone aux fins d'utilisation comme substance active pharmaceutique.
3. 1-(p-méthoxybenzoyl)-2-pyrrolidinone aux fins d'utilisation dans le traitement ou la prévention de l'insuffisance cérébrale ou dans l'amélioration de la capacité intellectuelle.
4. Procédé de préparation de 1-(p-méthoxybenzoyl)-2-pyrrolidinone, caractérisé en ce que

a) on acyle de façon correspondante la 2-pyrrolidinone en position 1 ou
b) on méthyle la 1-(p-hydroxybenzoyl)-2-pyrrolidinone ou
c) on réduit un dérivé de 2-pyrrolinone de formule générale

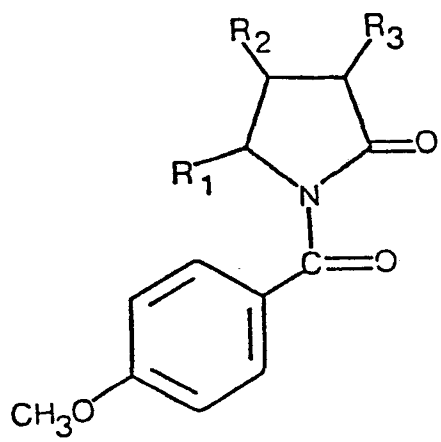

II

où l'un des radicaux $R^1$ et $R^3$ représente un hydrogène et l'autre forme avec $R^2$ une deuxième liaison C–C,